# EUROPEAN PATENT APPLICATION

(11) **EP 4 813 352 A1**
(43) Date of publication of application: **30.09.2026**
(21) Application number: 25165588.2
(22) Date of filing: 24.03.2025
(51) Int. Cl.: A61F 2/24

(54) **METHOD OF MANUFACTURING A HEMIVALVE PROSTHESIS**

(71) Applicant: AVVie GmbH, 1090 Wien (AT)
(72) Inventor: MOHL, Werner, 2571 Altenmarkt/Thennenberg (AT); KHIENWAD, Thananya, 1190n Wien (AT)
(74) Representative: SONN Patentanwälte GmbH & Co KG

(57) **Abstract**

A method of manufacturing a patient-specific hemivalve prosthesis for mitral valve repair comprises segmenting a patient's mitral valve from medical imaging data to generate a segmented mitral valve model, creating an anterior leaflet model and a posterior leaflet model from the segmented mitral valve model by determining geometric references including a commissure-to-commissure line between two commissures, a coaptation line based on overlapping surfaces between the leaflet models, a posterior annulus connecting points along the posterior leaflet model adjacent to the left atrial wall, and a free edge line along points of the anterior leaflet in a ventricle underneath an annulus plane, reconstructing the posterior leaflet model by creating a neo-coaptation line to achieve a minimum target coaptation height, and forming the hemivalve prosthesis based on the reconstructed posterior leaflet model such that the prosthesis is configured to establish a coaptation plane with the patient's anterior leaflet.

## Description

The present invention relates generally to cardiac valve repair devices and methods. More specifically, the invention relates to devices and methods for repairing mitral valve regurgitation through a transcatheter approach using imaging-guided prosthesis sizing and placement.

The mitral valve is one of four heart valves that direct blood flow through the heart. It is located between the left atrium and the left ventricle and functions to prevent the backflow of blood from the left ventricle into the left atrium during heart contraction. The mitral valve includes two leaflets, the anterior leaflet and the posterior leaflet, which are supported by a fibrous ring called the annulus and attached to papillary muscles in the left ventricle by chordae tendineae.

Mitral regurgitation (MR) occurs when the mitral valve fails to close properly, allowing blood to flow backwards from the left ventricle to the left atrium during systole. MR can be classified into two main categories: degenerative mitral regurgitation (DMR) and functional mitral regurgitation (FMR).

In DMR, there is a structural abnormality of the valve apparatus itself, typically involving the loss of chordal support that prevents proper coaptation between the leaflets. The uncompromised leaflet cannot properly coapt with the diseased leaflet, leading to prolapse and regurgitation.

In FMR, while the valve apparatus itself may be structurally normal, the left ventricle is enlarged, causing the chordae tendineae to pull the leaflets into the ventricle. This is particularly pronounced in the posterior leaflet, creating a closure gap that leads to severe insufficiency and retrograde flow.

Current surgical approaches for treating mitral regurgitation include various repair techniques. In DMR, surgical repair typically involves resecting the prolapsing segment and decreasing the annular dimensions, then pushing the reconstructed segment against the anterior leaflet to establish a coaptation plane. For FMR, undersized ring annuloplasty is commonly employed to establish a coaptation plane. However, this approach has limitations due to the angle of the diseased leaflet closure, which can prevent the repair from being durable over time.

Less invasive transcatheter approaches have emerged as alternatives to open surgery, particularly for high-risk patients. Proper valve closure may be achieved by complete or partial replacement of the dysfunctional, native heart valve with an artificial heart valve prosthesis. The tissue-type heart valve prosthesis has flexible leaflets supported by a base structure that projects into the flow stream. Tissue-type valves show similar functions as native human heart valves and mimic their natural flexing action to coapt against each other.

A hemivalve prosthesis represents an approach to treating mitral valve regurgitation by replacing or augmenting only a portion of the valve apparatus, typically focusing on the posterior leaflet while preserving the native anterior leaflet. This partial replacement strategy aims to restore proper valve function by providing a new coaptation surface for the preserved native leaflet while minimizing disruption to the complex anatomical structures of the heart.

A recent development in hemivalve prosthesis technology is disclosed in EP4403136A1, which describes an implant comprising a frame with lower and upper frame sections. The lower frame section supports an artificial leaflet designed to cover a native leaflet, while the upper frame section supports a flexible structure that rests against the atrial wall. The device features two end sections that protrude circumferentially to rest against the atrial wall above the anterior annulus section and includes a flexible structure with two sheets forming a blood-fillable cavity to prevent paravalvular leakage.

WO 2023086904 A1 discloses a prosthetic mitral valve designed to replace a portion of the native posterior scallops while preserving the remaining portions of the native posterior leaflets. The device features a frame carrying prosthetic leaflets and arms extending from the structural frame, with anchoring regions designed to interface with the native trigones.

Prior attempts to utilize hemivalve prostheses for mitral valve repair face several challenges. These include difficulty in achieving proper sizing and positioning due to the complex three-dimensional anatomy of the mitral valve, limited ability to address specific pathology in both DMR and FMR cases, and challenges in establishing adequate and durable coaptation between leaflets. Additional complications include the risk of systolic anterior motion of the anterior leaflet and limited durability of repairs due to ongoing ventricular remodeling in FMR cases.

Accordingly, the present invention aims to provide improved devices and methods for mitral valve repair that overcome the limitations of existing approaches.

More specifically, the present invention aims to provide a method for precise sizing and positioning of a mitral valve repair prosthesis based on patient-specific imaging data comprising a leaflet surface to securely coapt with the native valve leaflet. The invention further seeks to create a repair approach that respects the diseased mitral valve's natural anatomy and pathological changes while establishing a stable coaptation plane between the anterior leaflet and a prosthetic posterior leaflet component.

In order to solve these objects, the present invention provides a method of manufacturing a hemivalve prosthesis for mitral valve repair as defined in claim 1. The method begins with segmenting a patient's mitral valve from medical imaging data to generate a segmented mitral valve model. From this model, both an anterior leaflet model and a posterior leaflet model are created. The posterior leaflet model is created through several steps: determining a commissure-to-commissure line between two commissures, defining a coaptation line based on overlapping surfaces between the anterior leaflet model and the posterior leaflet model, establishing a posterior annulus by connecting points along the posterior leaflet model adjacent to the left atrial wall, and creating a free edge line along points of the anterior leaflet in a ventricle underneath an annulus plane. The method then involves reconstructing the posterior leaflet model by creating a neo-coaptation line in order to achieve a minimum target coaptation height. Finally, the hemivalve prosthesis is formed based on the reconstructed posterior leaflet model and is configured to establish a coaptation plane with the patient's anterior leaflet.

By utilizing patient-specific imaging data to generate the segmented valve model, the resulting prosthesis can be precisely tailored to the individual patient's anatomy. This personalization is particularly important given the significant anatomical variations between patients and the different pathological changes that can occur in mitral valve disease.

The posterior leaflet model is created by taking relevant anatomical features into consideration that affect valve function. By specifically defining the commissure-to-commissure line, coaptation line, posterior annulus, and free edge line, the method captures important geometric relationships that achieve successful modelling of the leaflets.

After the step of creating the anterior and posterior leaflet models from the segmented mitral valve model, these initial models represent the pathological state of the patient's mitral valve. Specifically, the models exhibit insufficient coaptation height between the anterior and posterior leaflets, which is the underlying cause of mitral regurgitation in the patient's valve.

The reconstruction of the posterior leaflet model according to the invention is therefore carried out with the specific purpose of changing the geometry and/or dimensions of the posterior leaflet so as to achieve a sufficient coaptation height with the anterior leaflet. This reconstruction transforms the model from one that reflects the pathological valve state to one that will restore proper valve function. By creating a neo-coaptation line with modified geometry, the reconstruction ensures that the prosthesis, when manufactured according to the reconstructed model, will establish adequate contact with the patient's anterior leaflet. In this way, the invention directly addresses the root cause of mitral regurgitation - insufficient coaptation - rather than merely attempting to modify the annular dimensions as in conventional repair methods.

Another aspect of this reconstruction process is the establishment of a proper tenting angle, which is defined as the angle between the annulus plane and the coaptation length. In pathological states, particularly in functional mitral regurgitation, this angle is frequently narrowed, contributing to inadequate valve closure. The reconstruction method according to the invention specifically addresses this by establishing a tenting angle that corresponds to that of a healthy valve. This geometric correction is a direct consequence of establishing sufficient coaptation height, as the proper positioning of the neo-coaptation line necessarily results in the formation of an appropriate tenting angle.

Based on the reconstructed posterior leaflet model, the hemivalve prosthesis is formed as a physical device that will replace the patient's posterior leaflet while working in conjunction with the patient's native anterior leaflet. The prosthesis is manufactured to embody the geometric specifications determined during the reconstruction step, particularly the neo-coaptation line that was designed to achieve sufficient coaptation height. When implanted, the prosthesis is configured to establish a coaptation plane with the patient's anterior leaflet, thereby restoring proper valve function. The physical prosthesis may be formed using suitable biocompatible materials through various manufacturing techniques such as molding, 3D printing, or other fabrication methods known in the art for producing cardiac valve prostheses.

According to a preferred embodiment of the invention, the reconstruction of the posterior leaflet model establishes a neo-coaptation line that achieves a minimum target coaptation height between 4 and 6 millimeters. This specific range of coaptation height has been found to provide optimal results in ensuring proper valve closure while preventing complications.

According to another preferred embodiment of the invention, the reconstruction of the posterior leaflet model includes specific steps to account for the natural segmentation of the posterior leaflet. In particular, the posterior annulus, coaptation line, and free edge lines are divided into three segments corresponding to the P1, P2, and P3 scallops of the native posterior leaflet. For each of these scallops, coaptation points are established based on the initial model. These coaptation points are then adjusted to achieve the minimum target coaptation height, and the neo-coaptation line is created by connecting these adjusted coaptation points. This segment-specific approach ensures that the reconstructed posterior leaflet model, and consequently the hemivalve prosthesis, maintains anatomically appropriate relationships between the P1, P2, and P3 portions while achieving the desired coaptation with the anterior leaflet. The adjustment of coaptation points for each scallop allows for precise control over the coaptation geometry across the entire posterior leaflet segment, contributing to optimal valve function.

According to yet another preferred embodiment of the invention, the division of the posterior annulus, coaptation line, and free edge lines into three segments corresponding to P1, P2, and P3 scallops is performed according to predetermined width ratios. In particular, these segments are preferably divided according to a width ratio of approximately 1:1.5:1, corresponding to P1:P2:P3 respectively. This specific ratio has been found to correspond to the natural geometric relationships observed in normal mitral valve anatomy, as established through anatomical studies. By incorporating these predetermined width ratios into the reconstruction process, the invention ensures that the resulting hemivalve prosthesis maintains physiologically appropriate proportions between the P1, P2, and P3 segments.

According to a further preferred embodiment of the invention, the medical imaging data used for segmenting the patient's mitral valve comprises computed tomography (CT) images that are aligned in multiple planes. Specifically, the CT images include coronal, sagittal, and axial views of the mitral valve apparatus. The multiplanar reformation planes are preferably aligned on the commissure-to-commissure line and the anterior-posterior plane in the short axis view, on the annulus plane in the coronal view, and on the commissure-to-commissure plane in the sagittal view. This multi-plane alignment is particularly advantageous as it enables accurate segmentation of the mitral valve model by providing complementary views of the valve structure.

Alternatively, the medical imaging data used for segmenting the patient's mitral valve may be obtained from at least one of computed tomography (CT), nuclear magnetic resonance (NMR), or echocardiography imaging. Each of these imaging modalities offers distinct advantages for analyzing the mitral valve structure. CT imaging provides high spatial resolution and excellent visualization of the valve apparatus and surrounding cardiac structures. NMR imaging offers superior soft tissue contrast and the ability to assess valve function dynamically. Echocardiography, particularly three-dimensional echocardiography, enables real-time visualization of valve motion and blood flow patterns. The method according to the invention can accommodate imaging data from any of these modalities, either individually or in combination, to generate the segmented mitral valve model.

According to still another preferred embodiment of the invention, the method further comprises determining a tenting angle between the annulus plane and a coaptation length for each reconstructed segment (P1, P2, P3) of the posterior leaflet model. Specifically, this tenting angle (α) is measured at the respective coaptation points by calculating the angle between the annulus plane and the coaptation length of each scallop.

According to yet another preferred embodiment of the invention, the method further comprises specific steps to account for papillary muscle interaction. In particular, the method includes measuring distances between papillary muscle tips and a midpoint of the mitral annulus. These measurements specifically include determining the distance between the anterolateral papillary muscle (ALPM) tip and the midpoint of the mitral annulus, as well as the distance between the posteromedial papillary muscle (PMPM) tip and the midpoint of the mitral annulus. Additionally, distances to the mitral annular plane are measured for both papillary muscles. These measurements are advantageously obtained using sagittal CT images, which offer a clearer view of the papillary muscle tips and the mitral annulus. Based on these measured distances, the position of the hemivalve prosthesis is determined to minimize potential interference with the papillary muscles while maintaining proper valve function.

According to still another preferred embodiment of the invention, the method further comprises determining specific angles to optimize anchoring of the hemivalve prosthesis in the left atrium. In particular, the angles between the annulus plane and the left atrial wall are determined at three locations: the anterior commissure, the posterior commissure, and the mid-posterior leaflet. By determining these specific angles between the annulus plane and the left atrial wall, the method enables the design of anchoring features that conform to the patient's unique left atrial anatomy.

According to further embodiments of the invention, the hemivalve prosthesis is manufactured to comprise a support structure configured to hold and position the reconstructed artificial posterior leaflet. The support structure may be formed as a frame having an annular or curved portion that follows the contour of the posterior mitral annulus and preferably extends upwards into the left atrium. This frame preferably includes anchoring elements designed to engage with the left atrial wall and/or the annulus at specific locations corresponding to the previously determined angles between the annulus plane and left atrial wall at the anterior commissure, posterior commissure, and mid-posterior region.

The reconstructed artificial posterior leaflet may be attached to the support structure using various biocompatible attachment methods, such as suturing, bonding, or integration during the manufacturing process. The leaflet material may comprise processed tissue or biocompatible synthetic materials having appropriate mechanical properties to withstand the repetitive stresses of cardiac cycling while maintaining proper coaptation with the native anterior leaflet. Suitable processed biological tissues may include bovine or porcine pericardium, which can be chemically treated to enhance durability while maintaining appropriate mechanical properties. Alternatively, synthetic materials such as expanded polytetrafluoroethylene (ePTFE), polyester fabric, or other biocompatible polymers may be employed.

The support structure may further comprise commissural posts at both ends, configured to align with the natural commissures of the mitral valve. These posts help maintain proper alignment of the prosthesis and contribute to the establishment of proper coaptation geometry. The frame may also include features specifically designed to prevent rotation or displacement of the prosthesis once implanted.

Alternatively, instead of using vertical posts connected to the leaflet, the reconstructed neo-posterior leaflet may comprise neo-chordae structures that extend from the neo-leaflet itself and are designed to be attached at the intersection point where the commissures meet the lower portion of the frame. This arrangement provides additional support and stability to the neo-leaflet structure while mimicking the natural chordal support found in the native mitral valve.

For fixation to the native mitral valve apparatus, the support structure may incorporate multiple anchoring mechanisms. These may include atrial fixation elements that extend into the left atrium, as well as annular fixation means that engage with the native posterior annulus. The specific geometry and positioning of these fixation elements are determined based on the previously measured anatomical parameters, including the angles between the annulus plane and left atrial wall.

According to further embodiments of the invention, the support frame of the hemivalve prosthesis may be constructed from various biocompatible metals or alloys suitable for long-term implantation. Particularly preferred materials include nitinol (nickel-titanium alloy), which provides advantageous superelastic properties and shape memory characteristics that facilitate both delivery and deployment of the prosthesis. Alternative materials may include cobalt-chromium alloys, medical-grade stainless steel, or other biocompatible metals with appropriate strength and fatigue resistance properties. The frame may be manufactured using various techniques, such as laser cutting from a tube, wire forming, or additive manufacturing processes, to achieve the precise geometric specifications determined during the reconstruction process.

The frame structure may be designed with varying strut patterns and thicknesses to provide different mechanical properties in different regions. For example, the atrial anchoring portions may be designed with greater flexibility to conform to the left atrial anatomy, while the annular or curved portion may be constructed with greater radial strength to maintain proper positioning. The frame may also incorporate features such as eyelets or attachment points for securing the reconstructed posterior leaflet material.

According to yet another embodiment of the invention, the hemivalve prosthesis is configured for transcatheter delivery through a patient's vasculature. To enable such delivery, the prosthesis is designed to be collapsible to a compressed configuration that allows it to be loaded into a delivery catheter. The frame structure may incorporate specific features, such as engineered fold points or living hinges, that facilitate controlled and predictable collapse of the prosthesis. The reconstructed posterior leaflet material is mounted to the frame in a manner that allows it to fold without damage during compression and to reliably return to its designed configuration upon deployment. This reconstructed posterior leaflet material may be composed of various materials including pericardial tissue or structured polymers with different material properties, and may incorporate force retainers that extend from the leaflet as neo-chordae to connect with the frame, providing additional structural support and stability.

The delivery system may comprise a catheter with an outer diameter suitable for transseptal access to the left atrium. The prosthesis may be mounted onto an inner delivery catheter that includes mechanisms for controlled release and deployment of the device. These mechanisms may include features that allow for staged deployment, enabling verification of proper positioning before final release. The delivery system may also incorporate steerable segments to facilitate navigation through the patient's anatomy, particularly for achieving the previously determined bending curves from the inferior vena cava to the septum and from the septum to the mitral valve.

To facilitate precise positioning during deployment, the prosthesis and delivery system may include radiopaque markers at key locations corresponding to anatomical landmarks identified during the reconstruction process. The deployment sequence may be specifically designed to first establish atrial anchoring, followed by proper positioning at the annular level, with final deployment ensuring proper orientation of the reconstructed posterior leaflet for optimal coaptation with the native anterior leaflet. The delivery system may also allow for repositioning or recapture of the prosthesis prior to final release, should adjustment be necessary during the implantation procedure.

The present invention will be better understood from the following detailed description of exemplary embodiments when read in conjunction with the accompanying drawings.

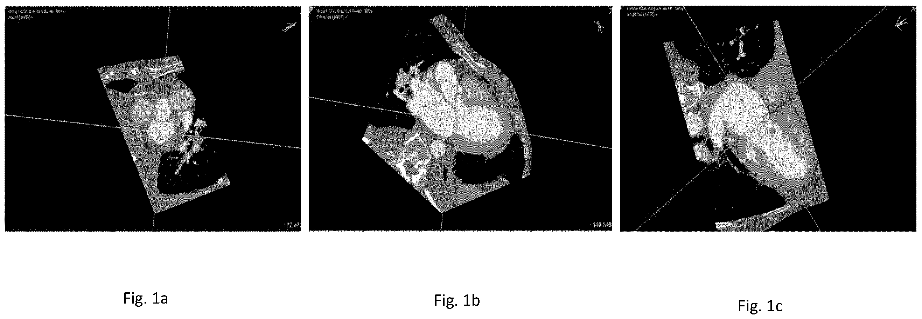

Fig. 1 a, b and c illustrate the segmentation of the mitral valve from multiple slices of CT images, including an axial, a coronal and a sagittal views.

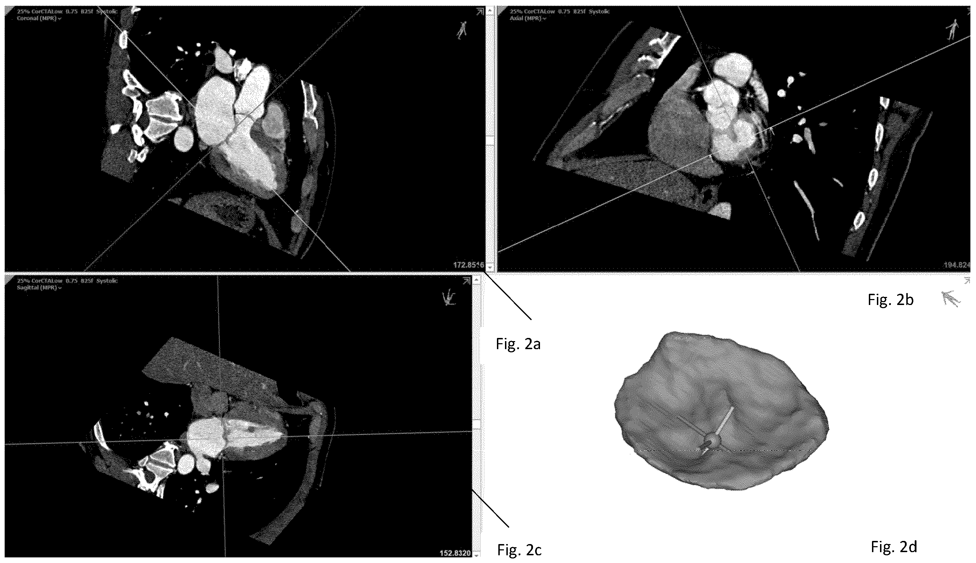

Fig. 2a-d illustrate the segmentation process of the mitral valve model from CT imaging data. The figure includes three CT image views and one 3D model, demonstrating the sequential steps of the segmentation process. Fig. 2a-c show how the muscle tissue thresholds in the CT images are carefully adjusted to isolate the relevant cardiac muscle structures, including the mitral valve area. The method begins in the coronal view, where the mitral valve region is manually delineated slice by slice. The process then addresses gaps between the individual slices through an interpolation technique to ensure continuity of the structure. The final refinement of the mitral valve model is achieved by incorporating additional anatomical information from both the axial and sagittal views, resulting in a comprehensive three-dimensional representation shown in Fig. 2d.

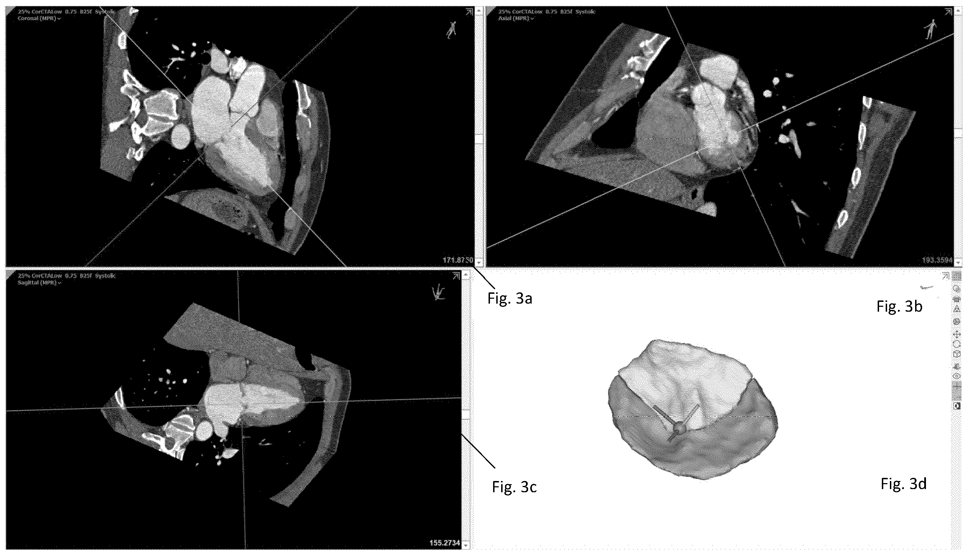

Fig. 3a-d illustrate the process of differentiating and segmenting the anterior leaflet and posterior leaflet. The figure comprises three CT image views and one 3D model demonstrating the segmentation workflow. In the coronal view (Fig. 3a), the P1, P2, and P3 sections of the posterior leaflet are initially delineated. Since the commissures between these segments are challenging to identify directly from the coronal view alone, the axis view (Fig. 3b) is employed to precisely locate the commissures by identifying the left and right sides of the fibrous trigone. Fig. 4c shows additional verification of the segmentation. Fig. 4d presents the final 3D model where the segmentation process has been completed, showing the posterior leaflet and the anterior leaflet as separate structures. This separation is achieved through a masking technique where the posterior leaflet is first masked, and then subtracted from the complete mitral valve mask to isolate the anterior leaflet portion, with the process being reversible to verify accuracy.

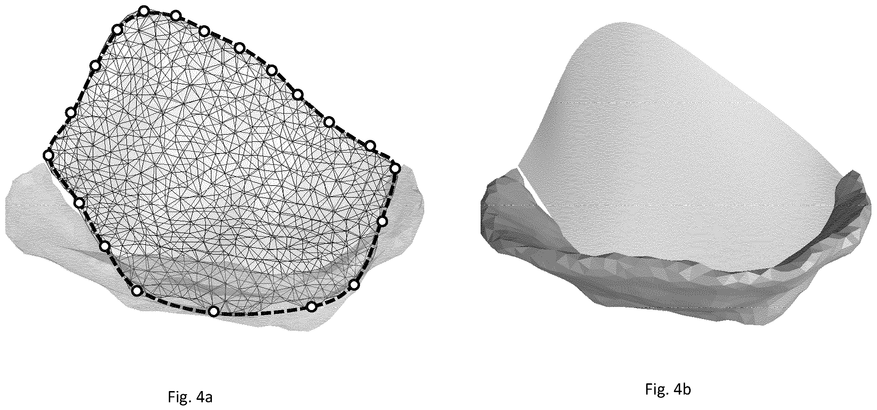

Fig. 4a and b show the process of creating the anterior leaflet surface from the segmented mitral valve model. Fig. 4a shows the initial segmented model with lines drawn to define the anterior leaflet circumference, where the model is depicted with a mesh overlay to show the structural detail. Fig. 4b demonstrates the final anterior leaflet surface that has been generated based on the defined circumference.

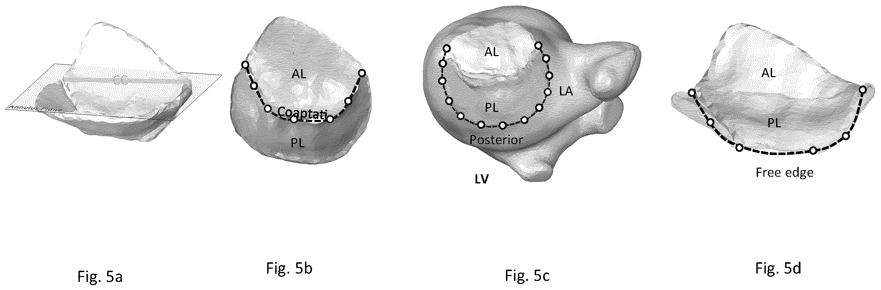

Fig. 5a-d shows a sequence of steps (1-4) for creating key geometric reference lines of the posterior leaflet model according to an embodiment of the invention. In step 1 (Fig. 5a), an annulus plane is established by connecting three cardinal points: the anterior commissure, the posterior commissure, and the mid-posterior annulus. The distance between the two commissures defines the commissure-to-commissure (CC) line, which serves as a primary reference dimension. Step 2 (Fig. 5b) illustrates the definition of the coaptation line, which is created by identifying multiple meeting points between the anterior leaflet (AL) and posterior leaflet (PL) where these structures naturally overlap. These meeting points are then connected to form a continuous coaptation line. In step 3 (Fig. 5c), the posterior annulus is defined by identifying and connecting a series of points along the posterior leaflet that lie adjacent to the left atrial (LA) wall. Finally, step 4 (Fig. 5d) depicts the creation of the free edge line, which is formed by connecting multiple points along the edge of the anterior leaflet that lie within the ventricle below the previously established annulus plane.

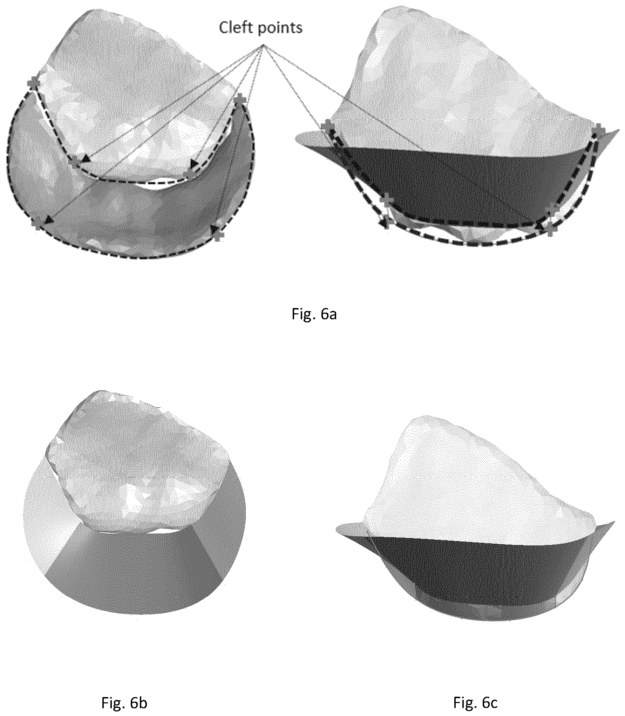

Fig. 6a-c illustrate the three-dimensional construction of the posterior leaflet model. The figures show progressive steps in defining the scallop geometry and coaptation surfaces. Fig. 6a demonstrates how predetermined width ratios of 1:1.5:1 are applied to divide the posterior leaflet into P1, P2, and P3 segments, with cleft points marked along the posterior annulus, coaptation line, and free edge lines. These ratios are based on established anatomical studies, as direct visualization of the scallops in CT imaging can be challenging. Fig. 6b shows the generation of coaptation length lines, which are created by connecting corresponding cleft points between the posterior annulus and the coaptation line, thereby defining the superior surfaces of the P1, P2, and P3 segments. Fig. 6c depicts the creation of additional connecting lines between segmented points on the coaptation line and free edge line, which serve to establish the coaptation height and define the specific coaptation areas for each of the P1, P2, and P3 segments.

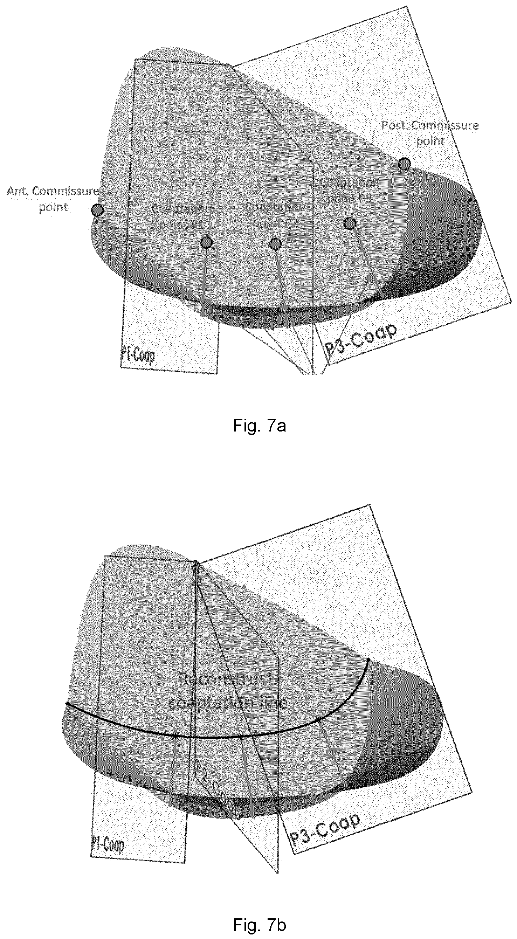

Fig. 7a and 7b illustrate the process of reconstructing the posterior leaflet coaptation. Fig. 7a demonstrates the establishment of new coaptation points, where five key points are identified: three new coaptation points (labeled as Coaptation point P1, P2, and P3) positioned at predetermined heights above the free edge of each scallop where they meet the anterior leaflet surface, plus two commissure points (labeled as Ant. Commissure point and Post. Commissure point). The positioning of these coaptation points is specifically determined to achieve coaptation heights derived from healthy mitral valve measurements found in literature studies. Importantly, these coaptation points are placed independent of both the cleft points between scallops and the centers of the bulging areas of the scallops. Fig. 7b shows how these five points are connected to form a reconstructed coaptation line (labeled as "Reconstruct coaptation line"), which is designed to establish sufficient contact between the anterior and posterior leaflets, thereby reducing mitral regurgitation.

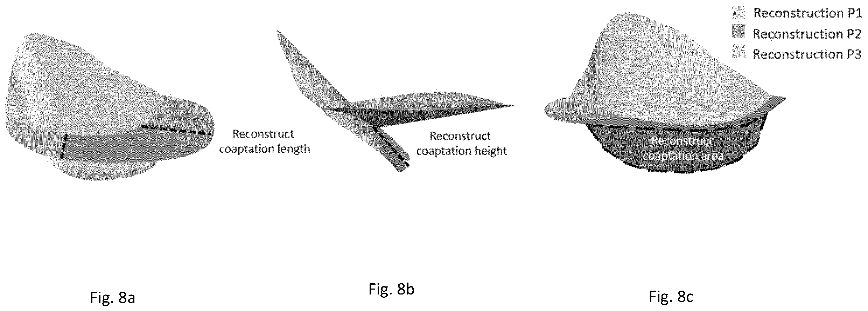

Fig. 8a-c demonstrates the creation and properties of the reconstructed coaptation surface. The figure includes three views showing different aspects of the reconstructed posterior leaflet geometry. Fig. 8a illustrates the creation of a new coaptation surface that is generated based on the previously established reconstructed coaptation line. Fig. 8b and 8c depict how the reconstructed surface achieves a critical design parameter: the establishment of a coaptation plane with a coaptation height measuring between 4 and 6 millimeters, oriented perpendicular to the annulus plane. This specific configuration is designed to simultaneously avoid movement obstruction and prevent kinking of the leaflet tissue. Further, it can be seen that the leaflet exhibits a controlled retroflection, wherein the angle formed by the flexed posterior reconstructed leaflet serves to prevent systolic anterior motion of the anterior leaflet, a potential complication in mitral valve repair procedures.

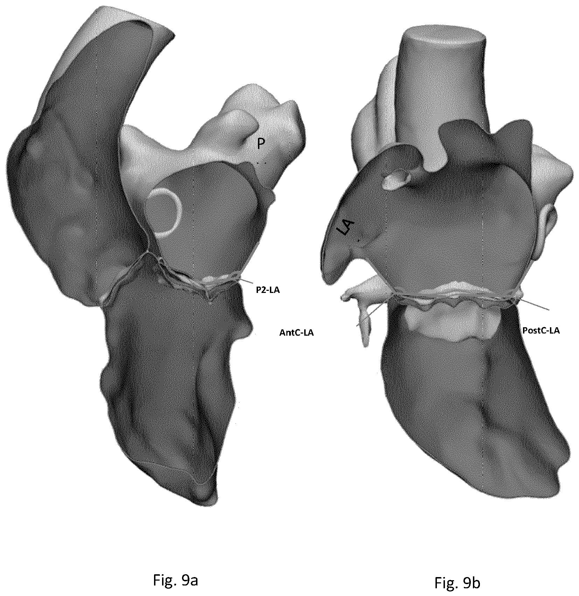

Fig. 9a and 9b illustrate the measurement of critical angles for optimizing left atrial anchoring. The figure presents both coronal and sagittal views of the left heart anatomy, specifically focusing on the interface between the mitral valve annulus and the left atrial wall. In both views, a reference line is shown at 20 millimeters above the annulus plane, indicating the height at which the measurements are taken. The coronal view demonstrates the measurement of the P2-LA angle, which is formed between the annulus plane and the left atrial wall at the mid-posterior leaflet region. The sagittal view shows the measurement of two additional angles: the AntC-LA angle at the anterior commissure and the PostC-LA angle at the posterior commissure. These measured angles between the annulus plane and the left atrial wall are parameters for designing the anchoring elements of the prosthesis.

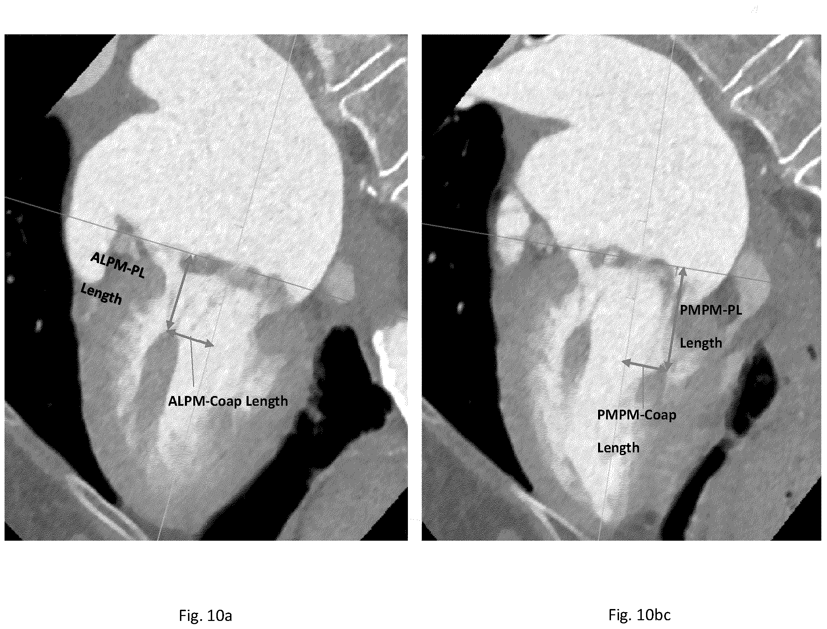

Fig. 10a and 10b illustrate the measurement of papillary muscle parameters n, shown through two comparative sagittal CT image views. Fig. 10a demonstrates the measurements related to the anterolateral papillary muscle (ALPM), while

Fig. 10b shows corresponding measurements for the posteromedial papillary muscle (PMPM). The images are specifically selected sagittal views that provide optimal visualization of the papillary muscle tips and their spatial relationship to the mitral annulus. In each panel, two critical measurements are indicated by bidirectional arrows: first, the distance between the respective papillary muscle tip and the midpoint of the mitral annulus (labeled as "ALPM-Coap Length" and "PMPM-Coap Length" respectively), and second, the distance between each papillary muscle and the mitral annular plane (labeled as "ALPM-PL Length" and "PMPM-PL Length" respectively). These measurements are for determining the optimal positioning of the hemivalve prosthesis to prevent interference with the native subvalvular apparatus while maintaining proper valve function.

## Claims

**1.** A method of manufacturing a hemivalve prosthesis for mitral valve repair, comprising:
segmenting a patient's mitral valve from medical imaging data to generate a segmented mitral valve model;
creating an anterior leaflet model from the segmented mitral valve model;
creating a posterior leaflet model from the segmented mitral valve model by :
determining a commissure-to-commissure line between two commissures;
defining a coaptation line based on overlapping surfaces between the anterior leaflet model and the posterior leaflet model;
establishing a posterior annulus by connecting points along the posterior leaflet model adjacent to the left atrial wall;
creating a free edge line along points of the anterior leaflet in a ventricle underneath an annulus plane;
reconstructing the posterior leaflet model by creating a neo-coaptation line in order to achieve a minimum target coaptation height; and
forming the hemivalve prosthesis based on the reconstructed posterior leaflet model, wherein the prosthesis is configured to establish a coaptation plane with the patient's anterior leaflet.

**2.** The method according to claim 1, wherein reconstructing the posterior leaflet model comprises:
dividing the posterior annulus, coaptation line, and free edge lines into three segments corresponding to P1, P2, and P3 scallops;
establishing coaptation points for each of the P1, P2, and P3 scallops;
adjusting the coaptation points and creating the neo-coaptation line by connecting the adjusted coaptation points.

**3.** The method according to claim 2, wherein dividing the posterior annulus, coaptation line, and free edge lines into three segments is performed according to predetermined width ratios, wherein the predetermined width ratios preferably are approximately 1:1.5:1.

**3.** The method according to claim 1, 2 or 3, wherein the medical imaging data comprises computed tomography (CT) images aligned in multiple planes including coronal, sagittal, and axial views.

**4.** The method according to any one of claims 1 to 3, further comprising determining a tenting angle between the annulus plane and a coaptation length for each segment of the posterior leaflet model.

**5.** The method according to any one of claims 1 to 4, further comprising: measuring a distance between papillary muscle tips and a midpoint of a mitral annulus; and determining a position of the hemivalve prosthesis based on the measured distance to minimize interaction with the papillary muscles.

**6.** The method according to any one of claims 1 to 5, further comprising determining angles between an annulus plane and a left atrial wall at an anterior commissure, posterior commissure, and mid-posterior leaflet for anchoring the hemivalve prosthesis in the left atrium.

**7.** The method according to any one of claims 1 to 6, wherein the medical imaging data is obtained from at least one of computed tomography (CT), nuclear magnetic resonance (NMR), or echocardiography imaging.

**8.** The method according to any one of claims 1 to 7, comprising creating a coaptation plane of at least 4mm between both commissures as a perpendicular plane between the annulus plane
